# EUROPEAN PATENT APPLICATION

(11) **EP 3 290 437 A1**
(43) Date of publication of application: **07.03.2018**
(21) Application number: 16186474.9
(22) Date of filing: 31.08.2016
(51) Int. Cl.: C07K 14/33, A61K 38/16, A61Q 19/00, C12N 9/52

(54) **NOVEL RECOMBINANT CLOSTRIDIAL NEUROTOXINS WITH DECREASED DURATION OF EFFECT**

(71) Applicant: Merz Pharma GmbH & Co. KGaA, 60318 Frankfurt am Main (DE)
(72) Inventor: Scheps, Daniel, 14471 Potsdam (DE); López de la Paz, Manuela, 65835 Liederbach am Taunus (DE); Hofmann, Fred, 14480 Potsdam (DE); Frevert, Jürgen, 10625 Berlin (DE)
(74) Representative: Graf Keyserlingk, Nikolai

(57) **Abstract**

This invention relates to novel recombinant clostridial neurotoxins exhibiting decreased duration of effect and to methods for the manufacture of such recombinant clostridial neurotoxins. These novel recombinant clostridial neurotoxins comprise a domain consisting of proline, valine and threonine residues, and the methods comprise the steps of inserting a nucleic acid sequence coding for said domain into a nucleic acid sequence coding for a parental clostridial neurotoxin and expression of the recombinant nucleic acid sequence comprising said domain-coding sequence in a host cell. The invention further relates to novel recombinant single-chain precursor clostridial neurotoxins used in such methods, nucleic acid sequences encoding such recombinant single-chain precursor clostridial neurotoxins, and pharmaceutical compositions comprising the recombinant clostridial neurotoxin with decreased duration of effect.

## Description

### FIELD OF THE INVENTION

This invention relates to novel recombinant clostridial neurotoxins exhibiting decreased duration of effect and to methods for the manufacture of such recombinant clostridial neurotoxins. These novel recombinant clostridial neurotoxins comprise a domain consisting of proline, valine and threonine residues, and the methods comprise the steps of inserting a nucleic acid sequence coding for said domain into a nucleic acid sequence coding for a parental clostridial neurotoxin and expression of the recombinant nucleic acid sequence comprising the domain-coding sequence in a host cell. The invention further relates to novel recombinant single-chain precursor clostridial neurotoxins used in such methods, nucleic acid sequences encoding such recombinant single-chain precursor clostridial neurotoxins, and pharmaceutical compositions comprising the recombinant clostridial neurotoxin with decreased duration of effect.

### BACKGROUND OF THE INVENTION

Clostridium is a genus of anaerobe gram-positive bacteria, belonging to the Firmicutes. Clostridium consists of around 100 species that include common free-living bacteria as well as important pathogens, such as *Clostridium botulinum* and *Clostridium tetani.* Both species produce neurotoxins, botulinum toxin and tetanus toxin, respectively. These neurotoxins are potent inhibitors of calcium-dependent neurotransmitter secretion of neuronal cells and are among the strongest toxins known to man. The lethal dose in humans lies between 0.1 ng and 1 ng per kilogram of body weight.

Oral ingestion of botulinum toxin via contaminated food or generation of botulinum toxin in wounds can cause botulism, which is characterised by paralysis of various muscles. Paralysis of the breathing muscles can cause death of the affected individual.

Although both botulinum neurotoxin (BoNT) and tetanus neurotoxin (TxNT) function via a similar initial physiological mechanism of action, inhibiting neurotransmitter release from the axon of the affected neuron into the synapse, they differ in their clinical response. While the botulinum toxin acts at the neuromuscular junction and other cholinergic synapses in the peripheral nervous system, inhibiting the release of the neurotransmitter acetylcholine and thereby causing flaccid paralysis, the tetanus toxin acts mainly in the central nervous system, preventing the release of the inhibitory neurotransmitters GABA (gamma-aminobutyric acid) and glycine by degrading the protein synaptobrevin. The consequent overactivity in the muscles results in generalized contractions of the agonist and antagonist musculature, termed a tetanic spasm (rigid paralysis).

While the tetanus neurotoxin exists in one immunologically distinct type, the botulinum neurotoxins are known to occur in seven different immunogenic types, termed BoNT/A through BoNT/H. Most *Clostridium botulinum* strains produce one type of neurotoxin, but strains producing multiple toxins have also been described.

Botulinum and tetanus neurotoxins have highly homologous amino acid sequences and show a similar domain structure. Their biologically active form comprises two peptide chains, a light chain of about 50 kDa and a heavy chain of about 100 kDa, linked by a disulfide bond. A linker or loop region, whose length varies among different clostridial toxins, is located between the two cysteine residues forming the disulfide bond. This loop region is proteolytically cleaved by an unknown clostridial endoprotease to obtain the biologically active toxin.

The molecular mechanism of intoxication by TeNT and BoNT appears to be similar as well: entry into the target neuron is mediated by binding of the C-terminal part of the heavy chain to a specific cell surface receptor; the toxin is then taken up by receptor-mediated endocytosis. The low pH in the so formed endosome then triggers a conformational change in the clostridial toxin which allows it to embed itself in the endosomal membrane and to translocate through the endosomal membrane into the cytoplasm, where the disulfide bond joining the heavy and the light chain is reduced. The light chain can then selectively cleave so called SNARE-proteins, which are essential for different steps of neurotransmitter release into the synaptic cleft, e.g. recognition, docking and fusion of neurotransmitter-containing vesicles with the plasma membrane. TeNT, BoNT/B, BoNT/D, BoNT/F, and BoNT/G cause proteolytic cleavage of synaptobrevin or VAMP (vesicle-associated membrane protein), BoNT/A and BoNT/E cleave the plasma membrane-associated protein SNAP-25, and BoNT/C cleaves the integral plasma membrane protein syntaxin and SNAP-25.

Clostridial neurotoxins display variable durations of action that are serotype specific. The clinical therapeutic effect of BoNT/A lasts approximately 3 months for neuromuscular disorders and 6 to 12 months for hyperhidrosis. The effect of BoNT/E, on the other hand, lasts less than 4 weeks. The longer lasting therapeutic effect of BoNT/A makes it preferable for certain clinical use compared to the other serotypes, for example serotypes B, C₁, D, E, F, G. One possible explanation for the divergent durations of action might be the distinct subcellular localizations of BoNT serotypes. The protease domain of BoNT/A light chain localizes in a punctate manner to the plasma membrane of neuronal cells, co-localizing with its substrate SNAP-25. In contrast, the short-duration BoNT/E serotype is cytoplasmic. Membrane association might protect BoNT/A from cytosolic degradation mechanisms allowing for prolonged persistence of BoNT/A in the neuronal cell.

In *Clostridium bofulinum,* the botulinum toxin is formed as a protein complex comprising the neurotoxic component and non-toxic proteins. The accessory proteins embed the neurotoxic component thereby protecting it from degradation by digestive enzymes in the gastrointestinal tract. Thus, botulinum neurotoxins of most serotypes are orally toxic. Complexes with, for example, 450 kDa or with 900 kDa are obtainable from cultures of *Clostridium botulinum.*

In recent years, botulinum neurotoxins have been used as therapeutic agents in the treatment of dystonias and spasms. Preparations comprising botulinum toxin complexes are commercially available, e.g. from Ipsen Ltd (Dysport^{®}) or Allergan Inc. (Botox^{®}). A high purity neurotoxic component, free of any complexing proteins, is for example available from Merz Pharmaceuticals GmbH, Frankfurt (Xeomin^{®}).

Clostridial neurotoxins are usually injected into the affected muscle tissue, bringing the agent close to the neuromuscular end plate, i.e. close to the cellular receptor mediating its uptake into the nerve cell controlling said affected muscle. Various degrees of neurotoxin spread have been observed. The neurotoxin spread is thought to depend on the injected amount and the particular neurotoxin preparation. It can result in adverse side effects such as paralysis in nearby muscle tissue, which can largely be avoided by reducing the injected doses to the therapeutically relevant level. Overdosing can also trigger the immune system to generate neutralizing antibodies that inactivate the neurotoxin preventing it from relieving the involuntary muscle activity. Immunologic tolerance to botulinum toxin has been shown to correlate with cumulative doses.

At present, clostridial neurotoxins are still predominantly produced by fermentation processes using appropriate Clostridium strains. However, industrial production of clostridial neurotoxin from anaerobic Clostridium culture is a cumbersome and time-consuming process. Due to the high toxicity of the final product, the procedure must be performed under strict containment. During the fermentation process, the single-chain precursors are proteolytically cleaved by an unknown clostridial protease to obtain the biologically active di-chain clostridial neurotoxin. The degree of neurotoxin activation by proteolytic cleavage varies between different strains and neurotoxin serotypes, which is a major consideration for the manufacture due to the requirement of neurotoxin preparations with a well-defined biological activity. Furthermore, during fermentation processes using Clostridium strains the clostridial neurotoxins are produced as protein complexes, in which the neurotoxic component is embedded by accessory proteins. These accessory proteins have no beneficial effect on biological activity or duration of effect. They can however trigger an immune reaction in the patient, resulting in immunity against the clostridial neurotoxin. Manufacture of recombinant clostridial neurotoxins, which are not embedded by auxiliary proteins, might therefore be advantageous.

Methods for the recombinant expression of clostridial neurotoxins in E. *coli* are well known in the art (see, for example, WO 00/12728, WO 01/14570, or WO 2006/076902). Furthermore, clostridial neurotoxins have been expressed in eukaryotic expression systems, such as in *Pichia pastoris, Pichia methanolica, Saccharomyces cerevisiae,* insect cells and mammalian cells (see WO 2006/017749).

Recombinant clostridial neurotoxins may be expressed as single-chain precursors, which subsequently have to be proteolytically cleaved to obtain the final biologically active clostridial neurotoxin. Thus, clostridial neurotoxins may be expressed in high yield in rapidly-growing bacteria as relatively non-toxic single-chain polypeptides.

Furthermore, it might be advantageous to modify clostridial neurotoxin characteristics regarding biological activity, cell specificity, antigenic potential and duration of effect by genetic engineering to obtain recombinant neurotoxins with new therapeutic properties in specific clinical areas. Genetic modification of clostridial neurotoxins might allow altering the mode of action or expanding the range of therapeutic targets.

Botulinum toxin variants exhibiting a decreased duration of effect in neuromuscular tissue than naturally occurring botulinum toxins would be very advantageous for certain indications where a shortened duration of effect is desired.

US 2002/0127247 describes clostridial neurotoxins comprising modifications in secondary modification sites and exhibiting altered biological persistence. WO2011/000929 and WO 2013/068476 disclose modified clostridial neurotoxin with degradation signals in their light chain which reduces the duration of action. WO2013/017522 discloses a modified clostridial neurotoxin with altered duration of action due to an artificially introduced Calpain cleavage site.

There is a strong demand to produce new clostridial neurotoxins with a decreased duration of effect with improved properties, in order to allow for exploitation of the therapeutic potential of BoNT serotypes, which have so far been considered impractical for certain clinical application. Ideally, the decreased duration of effect of a particular clostridial neurotoxin could be adjusted in a tailor-made fashion in order to address any particular features and demands of a given indication, such as the amount of neurotoxin being administered, frequency of administration etc. In addition, it would be desirable to produce clostridial neurotoxins exhibit both i) a decreased duration of effect in neuromuscular tissue than naturally occurring botulinum toxins and ii) a high specific activity. To date, such aspects have not been solved satisfactorily.

### OBJECTS OF THE INVENTION

It was an object of the invention to provide recombinant clostridial neurotoxins exhibiting a decreased duration of effect in neuromuscular tissue than naturally occurring botulinum toxins. It was also an object of the invention to provide recombinant clostridial neurotoxins exhibiting both i) a decreased duration of effect in neuromuscular tissue than naturally occurring botulinum toxins and ii) a high specific activity. It was a further object of the invention to establish a reliable and accurate method for manufacturing and obtaining such recombinant clostridial neurotoxins. Such a method and novel precursor clostridial neurotoxins used in such methods would serve to satisfy the great need for recombinant clostridial neurotoxins exhibiting a decreased duration of effect.

### SUMMARY OF THE INVENTION

The naturally occurring botulinum toxin serotypes display highly divergent durations of effect, probably due to their distinct subcellular localization. BoNT/A exhibiting the longest persistence was shown to localize in the vicinity of the plasma membrane of neuronal cells, whereas the short-duration BoNT/E serotype is cytosolic. However, additional factors such as degradation, diffusion, and/or translocation rates might have a decisive impact on the differences in the duration of effect for the individual botulinum toxin serotypes.

So far, no generally applicable method for modifying clostridial neurotoxins in order to decrease their duration of effect is available. Surprisingly, it has been found that recombinant clostridial neurotoxins having such an effect can be obtained by cloning a sequence encoding a specific domain into a gene encoding a parental clostridial neurotoxin, and by subsequent heterologous expression of the generated construct in recombinant host cells, wherein this domain consists of proline, valine and threonine residues.

Thus, in one aspect, the present invention relates to recombinant clostridial neurotoxin comprising a domain, wherein this domain consists of proline, valine and threonine residues.

In another aspect, the present invention relates to a pharmaceutical composition comprising the recombinant clostridial neurotoxin of the present invention.

In yet another aspect, the present invention relates to the use of the composition of the present invention for cosmetic treatment.

In another aspect, the present invention relates to a method for the generation of the recombinant clostridial neurotoxin of the present invention, comprising the step of obtaining a recombinant nucleic acid sequence encoding a recombinant single-chain precursor clostridial neurotoxin by the insertion of a nucleic acid sequence encoding said domain into a nucleic acid sequence encoding a parental clostridial neurotoxin.

In another aspect, the present invention relates to a recombinant single-chain precursor clostridial neurotoxin comprising a domain consisting of proline, valine and threonine residues.

In another aspect, the present invention relates to a nucleic acid sequence encoding the recombinant single-chain precursor clostridial neurotoxin of the present invention.

In another aspect, the present invention relates to a method for obtaining the nucleic acid sequence of the present invention, comprising the step of inserting a nucleic acid sequence encoding said domain into a nucleic acid sequence encoding a parental clostridial neurotoxin.

In another aspect, the present invention relates to a vector comprising the nucleic acid sequence of the present invention, or the nucleic acid sequence obtainable by the method of the present invention.

In another aspect, the present invention relates to a recombinant host cell comprising the nucleic acid sequence of the present invention, the nucleic acid sequence obtainable by the method of the present invention, or the vector of the present invention.

In another aspect, the present invention relates to a method for producing the recombinant single-chain precursor clostridial neurotoxin of the present invention, comprising the step of expressing the nucleic acid sequence of the present invention, or the nucleic acid sequence obtainable by the method of the present invention, or the vector of the present invention in a recombinant host cell, or cultivating the recombinant host cell of the present invention under conditions that result in the expression of said nucleic acid sequence.

### FIGURES

**Figure 1****:** Schematic Presentation of a PVT Botulinum Toxin A (PVT-100-rBoNT/A).
**Figure 2****:** SDS·PAGE of purified PVT-rBoNT/A. Prior to applying the samples to the gel, ß-mercaptoethanol was added.
   Lane "v.V." (before activation): purified, non-activated single-chain PVT100-rBoNT/A having a molecular weight (Mw) of about 170 kDa.
   Lanes "n.A." (after activation) and "n.R." (after purification) show light chain (Lc-PVT-100-BoNT/A) and heavy chain (Hc) obtained after activation by thrombin under reducing conditions.

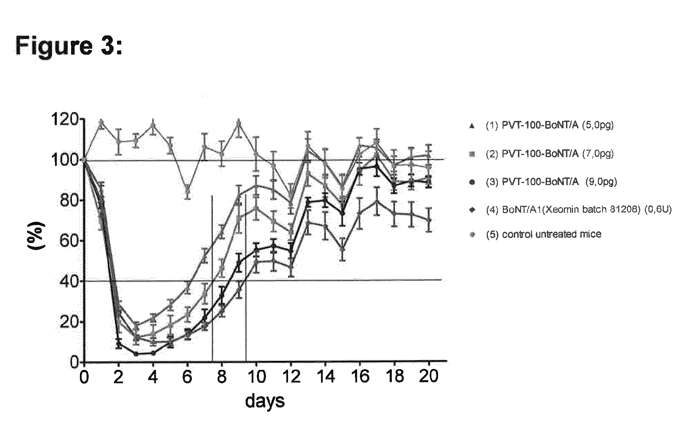

**Figure 3****:** Mouse running assay with PVT-100-rBoNT/A

### DETAILED DESCRIPTION OF THE INVENTION

The present invention may be understood more readily by reference to the following detailed description of the invention and the examples included therein.

In one aspect, the present invention relates to a recombinant clostridial neurotoxin comprising a domain consisting of proline, valine and threonine residues.

In the context of the present invention, the term "clostridial neurotoxin" refers to a natural neurotoxin obtainable from bacteria of the class Clostridia, including *Clostridium tetani* and *Clostridium botulinum,* or to a neurotoxin obtainable from alternative sources, including from recombinant technologies or from genetic or chemical modification. Particularly, the clostridial neurotoxins have endopeptidase activity.

Clostridial neurotoxins are produced as single-chain precursors that are proteolytically cleaved by an unknown clostridial endoprotease within the loop region to obtain the biologically active disulfide-linked di-chain form of the neurotoxin, which comprises two chain elements, a functionally active light chain and a functionally active heavy chain, where one end of the light chain is linked to one end of the heavy chain not via a peptide bond, but via a disulfide bond.

In the context of the present invention, the term "clostridial neurotoxin light chain" refers to that part of a clostridial neurotoxin that comprises an endopeptidase activity responsible for cleaving one or more proteins that is/are part of the so-called SNARE-complex involved in the process resulting in the release of neurotransmitter into the synaptic cleft: In naturally occurring clostridial neurotoxins, the light chain has a molecular weight of approx. 50 kDa.

In the context of the present invention, the term "clostridial neurotoxin heavy chain" refers to that part of a clostridial neurotoxin that is responsible for entry of the neurotoxin into the neuronal cell: In naturally occurring clostridial neurotoxins, the heavy chain has a molecular weight of approx. 100 kDa.

In the context of the present invention, the term "functionally active clostridial neurotoxin chain" refers to a recombinant clostridial neurotoxin chain able to perform the biological functions of a naturally occurring *Clostridium botulinum* neurotoxin chain to at least about 50%, particularly to at least about 60%, to at least about 70%, to at least about 80%, and most particularly to at least about 90%, where the biological functions of clostridial neurotoxin chains include, but are not limited to, binding of the heavy chain to the neuronal cell, entry of the neurotoxin into a neuronal cell, release of the light chain from the di-chain neurotoxin, and endopeptidase activity of the light chain. Methods for determining a neurotoxic activity can be found, for example, in WO 95/32738, which describes the reconstitution of separately obtained light and heavy chains of tetanus toxin and botulinum toxin. Also cell-based assay methods as described for example in WO2009/114748, WO 2013/049508 and WO2014/207109.

In the context of the present invention, the term "about" or "approximately" means within 20%, alternatively within 10%, including within 5% of a given value or range. Alternatively, especially in biological systems, the term "about" means within about a log (i.e. an order of magnitude), including within a factor of two of a given value.

In the context of the present invention, the term "recombinant clostridial neurotoxin" refers to a composition comprising a clostridial neurotoxin that is obtained by expression of the neurotoxin in a heterologous cell such as *E*. *coli,* and including, but not limited to, the raw material obtained from a fermentation process (supernatant, composition after cell lysis), a fraction comprising a clostridial neurotoxin obtained from separating the ingredients of such a raw material in a purification process, an isolated and essentially pure protein, and a formulation for pharmaceutical and/or aesthetic use comprising a clostridial neurotoxin and additionally pharmaceutically acceptable solvents and/or excipients.

In the context of the present invention, the term "recombinant clostridial neurotoxin" further refers to a clostridial neurotoxin based on a parental clostridial neurotoxin additionally comprising a heterologous domain wherein this domain consists of proline, valine and threonine residues, i.e. a domain that is not naturally occurring in said parental clostridial neurotoxin, in particular a synthetic domain, or a domain from a species other than *Clostridium botulinum,* in particular a domain from a human protein.

In the context of the present invention, the term "comprises" or "comprising" means "including, but not limited to". The term is intended to be open-ended, to specify the presence of any stated features, elements, integers, steps or components, but not to preclude the presence or addition of one or more other features, elements, integers, steps, components, or groups thereof. The term "comprising" thus includes the more restrictive terms "consisting of" and "consisting essentially of".

In the context of the present invention, the term "disordered state domain" refers to a protein segment, which is essentially lacking a secondary structure. Disordered state domains can be detected using a variety of methods, including spectroscopic methods such as circular dichroism or nuclear magnetic resonance (NMR) methods, including multidimensional NMR experiments, or crystallographic structure determinations.

In particular embodiments, said domain consisting of proline, valine and threonine residues comprises an amino acid sequence consisting of at least 50 amino acid residues, particularly between 50 and 3000 amino acid residues, more particularly between 60 and 500 amino acid residues, more particularly between 70 and 260 amino acid residues, more particularly between 80 and 240 amino acid residues, more particularly between 90 and 220 amino acid residues, particularly 100 amino acid residues, 150 amino acid residues, or 200 amino acid residues.

In particular embodiments, said domain consisting of proline, valine and threonine residues forms a disordered state domain.

Surprisingly, it has been found that the attachment of a domain consisting of proline, valine and threonine residues is able to shorten the duration of effect of a neurotoxin. A person skilled in the art would expect the opposite effect. It has been shown in WO2015/132004 that the attachment of a neurotoxin with Proline (P), Alanine (A) and Serine (S) residues extends the duration of effect. It has been speculated that the spread of a botulinum toxin might be reduced by an attachment of a domain consisting of certain amino acids due to a larger hydrodynamic radius of the fusion protein and thus, more neurotoxin could reach the neuromuscular junctions which leads to a longer duration of effect. However, in the context of the present invention the attachment of a domain consisting of proline, valine and threonine residues surprisingly leads to a decreased duration of effect.

In particular embodiments, said domain comprises a plurality of amino acid repeats, wherein said repeat consist of Pro, Val, and Thr residues and wherein no more than six consecutive amino acid residues are identical.

In particular embodiments, the proline residues comprised in said domain constitute more than 4% and less than 40% of the amino acids of said domain.

In particular embodiments, said domain comprises at least the following amino acid sequence VTPVVPVPVTPVVPVPTVPV (SEQ ID NO: 1) or circular permuted versions or at least one multimer of the sequence as a whole or parts of the sequence, particularly (VTPVVPVPVTPVVPVPTVPV)ₙ, with n being an integer selected from 3 to 25, more particularly from 4 to 8, in particular wherein n is 5.

In particular embodiments, said domain is inserted at (i) the N-terminus of the light chain of said recombinant clostridial neurotoxin; (ii) the C-terminus of the light chain of said recombinant clostridial neurotoxin; (iii) the N-terminus of the heavy chain of said recombinant clostridial neurotoxin; or (iv) the C-terminus of the heavy chain of said recombinant clostridial neurotoxin.

In particular embodiments, the sequence of said clostridial neurotoxin is selected from the sequence of (i) a *Clostridium botulinum* neurotoxin serotype A, B, C, D, E, F, and G, particularly *Clostridium botulinum* neurotoxin serotype A, C and E, particularly *Clostridium botulinum* neurotoxin serotype A, or (ii) from the sequence of a functional variant of a *Clostridium botulinum* neurotoxin of (i), or (iii) from the sequence of a chimeric *Clostridium botulinum* neurotoxin, wherein the clostridial neurotoxin light chain and heavy chain are from different parental clostridial neurotoxin serotypes.

In the context of the present invention, the term *"Clostridium botulinum* neurotoxin serotype A, B, C, D, E, F, and G" refers to neurotoxins found in and obtainable from *Clostridium botulinum.* Currently, seven serologically distinct types, designated serotypes A, B, C, D, E, F, and G are known, including certain subtypes (e.g. A1, A2, A3, A4 and A5).

In particular embodiments the clostridial neurotoxin is selected from a *Clostridium botulinum* neurotoxin serotype A, C and E, in particular from *Clostridium botulinum* neurotoxin serotype A, or from a functional variant of any such *Clostridium botulinum* neurotoxin.

In particular embodiments, said recombinant clostridial neurotoxin has a light chain and a heavy chain comprised in the amino acid sequence as found in SEQ ID NO: 2.

In the context of the present invention, the term "functional variant of a clostridial neurotoxin" refers to a neurotoxin that differs in the amino acid sequence and/or the nucleic acid sequence encoding the amino acid sequence from a clostridial neurotoxin, but is still functionally active. In the context of the present invention, the term "functionally active" refers to the property of a recombinant clostridial neurotoxin to exhibit a biological activity of at least about 50%, particularly to at least about 60%, at least about 70%, at least about 80%, and most particularly at least about 90% of the biological activity of a naturally occurring parental clostridial neurotoxin, i.e. a parental clostridial neurotoxin without said domain, where the biological functions include, but are not limited to, binding to the neurotoxin receptor, entry of the neurotoxin into a neuronal cell, release of the light chain from the two-chain neurotoxin, and endopeptidase activity of the light chain, and thus inhibition of neurotransmitter release from the affected nerve cell. In vivo assays for assessing biological activity include the mouse LD50 assay and the ex vivo mouse hemidiaphragm assay as described by Pearce et al. (Pearce 1994, Toxicol. Appl. Pharmacol. 128: 69-77) and Dressler et al. (Dressler 2005, Mov. Disord. 20:1617-1619, Keller 2006, Neuroscience 139: 629-637) or a cell-based assay as described in WO2009/114748, WO2014/207109 or WO 2013/049508. The biological activity is commonly expressed in Mouse Units (MU). As used herein, 1 MU is the amount of neurotoxic component, which kills 50% of a specified mouse population after intraperitoneal injection, i.e. the mouse i.p. LD50.

On the protein level, a functional variant will maintain key features of the corresponding clostridial neurotoxin, such as key residues for the endopeptidase activity in the light chain, or key residues for the attachment to the neurotoxin receptors or for translocation through the endosomal membrane in the heavy chain, but may contain one or more mutations comprising a deletion of one or more amino acids of the corresponding clostridial neurotoxin, an addition of one or more amino acids of the corresponding clostridial neurotoxin, and/or a substitution of one or more amino acids of the corresponding clostridial neurotoxin. Particularly, said deleted, added and/or substituted amino acids are consecutive amino acids. According to the teaching of the present invention, any number of amino acids may be added, deleted, and/or substituted, as long as the functional variant remains biologically active. For example, 1, 2, 3, 4, 5, up to 10, up to 15, up to 25, up to 50, up to 100, up to 200, up to 400, up to 500 amino acids or even more amino acids may be added, deleted, and/or substituted. Accordingly, a functional variant of the neurotoxin may be a biologically active fragment of a naturally occurring neurotoxin. This neurotoxin fragment may contain an N-terminal, C-terminal, and/or one or more internal deletion(s).

In another embodiment, the functional variant of a clostridial neurotoxin additionally comprises a signal peptide. Usually, said signal peptide will be located at the N-terminus of the neurotoxin. Many such signal peptides are known in the art and are comprised by the present invention. In particular, the signal peptide results in transport of the neurotoxin across a biological membrane, such as the membrane of the endoplasmic reticulum, the Golgi membrane or the plasma membrane of a eukaryotic or prokaryotic cell. It has been found that signal peptides, when attached to the neurotoxin, will mediate secretion of the neurotoxin into the supernatant of the cells. In certain embodiments, the signal peptide will be cleaved off in the course of, or subsequent to, secretion, so that the secreted protein lacks the N-terminal signal peptide, is composed of separate light and heavy chains, which are covalently linked by disulfide bridges, and is proteolytically active.

In particular embodiments, the functional variant has in its clostridium neurotoxin part a sequence identity of at least about 40%, at least about 50%, at least about 60%, at least about 70% or most particularly at least about 80%, and a sequence homology of at least about 60%, at least about 70%, at least about 80%, at least about 90%, or most particularly at least about 95% to the corresponding part in the parental clostridial neurotoxin. Methods and algorithms for determining sequence identity and/or homology, including the comparison of variants having deletions, additions, and/or substitutions relative to a parental sequence, are well known to the practitioner of ordinary skill in the art. The term "identity" as used herein refers to sequence identity characterized by determining the number of identical amino acids between two nucleic acid sequences or two amino acid sequences wherein the sequences are aligned so that the highest order match is obtained. It can be calculated using published techniques or methods codified in computer programs such as, for example, BLASTP, BLASTN or FASTA (Altschul 1990, J Mol Biol 215, 403). The percent identity values are, in one aspect, calculated over the entire amino acid sequence. A series of programs based on a variety of algorithms is available to the skilled worker for comparing different sequences. In this context, the algorithms of Needleman and Wunsch or Smith and Waterman give particularly reliable results. To carry out the sequence alignments, the program PileUp (Higgins 1989, CABIOS 5, 151) or the programs Gap and BestFit (Needleman 1970, J Mol Biol 48; 443; Smith 1981, Adv Appl Math 2, 482), which are part of the GCG software packet (Genetics Computer Group 1991, 575 Science Drive, Madison, Wisconsin, USA 53711), may be used. The sequence identity values recited above in percent (%) are to be determined, in another aspect of the invention, using the program GAP over the entire sequence region with the following settings: Gap Weight: 50, Length Weight: 3, Average Match: 10.000 and Average Mismatch: 0.000, which, unless otherwise specified, shall always be used as standard settings for sequence alignments.On the DNA level, the nucleic acid sequences encoding the functional homologue and the parental clostridial neurotoxin may differ to a larger extent due to the degeneracy of the genetic code. It is known that the usage of codons is different between prokaryotic and eukaryotic organisms. Thus, when expressing a prokaryotic protein such as a clostridial neurotoxin, in a eukaryotic expression system, it may be necessary, or at least helpful, to adapt the nucleic acid sequence to the codon usage of the expression host cell, meaning that sequence identity or homology may be rather low on the nucleic acid level.

In the context of the present invention, the term "variant" refers to a neurotoxin that is a chemically, enzymatically, or genetically modified derivative of a corresponding clostridial neurotoxin, including chemically or genetically modified neurotoxin from *C. botulinum,* particularly of C. *botulinum* neurotoxin serotype A, C or E. A chemically modified derivative may be one that is modified by pyruvation, phosphorylation, sulfatation, lipidation, pegylation, glycosylation and/or the chemical addition of an amino acid or a polypeptide comprising between 2 and about 100 amino acids, including modification occurring in the eukaryotic host cell used for expressing the derivative. An enzymatically modified derivative is one that is modified by the activity of enzymes, such as endo- or exoproteolytic enzymes, including modification by enzymes of the eukaryotic host cell used for expressing the derivative. As pointed out above, a genetically modified derivative is one that has been modified by deletion or substitution of one or more amino acids contained in, or by addition of one or more amino acids (including polypeptides comprising between 2 and about 100 amino acids) to, the amino acid sequence of said clostridial neurotoxin. Methods for designing and constructing such chemically or genetically modified derivatives and for testing of such variants for functionality are well known to anyone of ordinary skill in the art.

In particular embodiments, said recombinant clostridial neurotoxin shows decreased duration of effect relative to an identical clostridial neurotoxin without the domain consisting of proline, valine and threonine residues.

In the context of the present invention, the term "decreased duration of effect" or "decreased duration of action" refers to a shorter lasting denervation mediated by a clostridial neurotoxin of the present invention. For example, as disclosed herein, administration of a disulfide-linked di-chain clostridial neurotoxin comprising a domain consisting of proline, valine and threonine residues results in localized paralysis for a shorter period of time relative to administration of an identical disulfide-linked di-chain clostridial neurotoxin without the coiled coil domain.

In the context of the present invention, the term "decreased duration of effect/action" is defined as a more than about 20%, particularly more than about 50%, more particularly more than about 90% decreased duration of effect of the recombinant neurotoxin of the present invention relative to the identical neurotoxin without the domain consisting of proline, valine and threonine residues.

In the context of the present invention the term "denervation" refers to denervation resulting from administration of a chemodenervating agent, for example a neurotoxin.

In the context of the present invention, the term "localized denervation" or "localized paralysis" refers to denervation of a particular anatomical region, usually a muscle or a group of anatomically and/or physiologically related muscles, which results from administration of a chemodenervating agent, for example a neurotoxin, to the particular anatomical region.

Without wishing to be bound by theory, the recombinant clostridial neurotoxins of the present invention might show decreased biological half-life, increased degradation rates, decreased uptake by neuronal cells, and/or modified intracellular translocation rates, in each case relative to an identical parental clostridial neurotoxin without the domain consisting of proline, valine and threonine residues.

In particular embodiments, the decreased duration of effect is due to a decreased biological half-life.

In the context of the present invention, the term "biological half-life" specifies the lifespan of a protein, for example of a clostridial neurotoxin, *in vivo.* In the context of the present invention, the term "biological half-life" refers to the period of time, by which half of a protein pool is degraded *in vivo.* For example it refers to the period of time, by which half of the amount of clostridial neurotoxin of one administered dosage is degraded.

In the context of the present invention, the term "decreased biological half-life" is defined as a more than about 20%, particularly more than about 50%, more particularly more than about 90% decreased biological half-life of the recombinant neurotoxin of the present invention relative to the identical neurotoxin without the domain consisting of proline, valine and threonine residues.

In the context of the present invention, the term "increased degradation rate" means that the domain consisting of proline, valine and threonine residues (PVT sequence) possibly enhances the degradation processes of the light chain in the cytosol of the neuron such as, for example, the attack of proteases or modifying enzymes like E3 ligases. Thus, the half-life of the light chain in the neuron is shortened resulting in a decreased duration of the therapeutic effect.

In particular embodiments of the invention, said recombinant clostridial neurotoxin comprising a domain consisting of proline, valine and threonine residues shows both i) a decreased duration of effect relative to an identical clostridial neurotoxin without said domain and ii) a high specific activity.

In the context of the present invention, the term "high specific activity" means that the specific activity is > 50 Units / ng neurotoxin. The specific neurotoxin activity is defined as the biologic activity (in units) per mass of neurotoxin protein (in ng protein).

In particular embodiments, the recombinant clostridial neurotoxin is for the use in the treatment of a disease requiring improved chemodenervation, wherein the recombinant clostridial neurotoxin causes shorter lasting denervation relative to an identical clostridial neurotoxin without the domain consisting of proline, valine and threonine residues.

In another aspect, the present invention relates to a pharmaceutical or cosmetic composition comprising the recombinant clostridial neurotoxin of the present invention. For preparing a pharmaceutical preparation comprising a clostridial neurotoxin the toxin can be formulated by various techniques dependent on the desired application purposes which are known in the art. For example, the (biologically active) botulinum neurotoxin polypeptide can be used in combination with one or more pharmaceutically acceptable carriers as a pharmaceutical composition. The pharmaceutically acceptable carrier(s) must be acceptable in the sense of being compatible with the other ingredients of the formulation and being not deleterious to the recipient thereof. The pharmaceutical carrier employed may include a solid, a gel, or a liquid. Exemplary of solid carriers are lactose, terra alba, sucrose, talc, gelatine, agar, pectin, acacia, magnesium stearate, stearic acid and the like. Exemplary of liquid carriers are glycerol, phosphate buffered saline solution, water, emulsions, various types of wetting agents, and the like. Suitable carriers comprise those mentioned above and others well known in the art, see, e.g., Remington's Pharmaceutical Sciences, Mack Publishing Company, Easton, Pennsylvania. In an aspect, the pharmaceutical composition can be dissolved in a diluent, prior to administration. The diluent is also selected so as not to affect the biological activity of the Neurotoxin product. Examples of such diluents are distilled water or physiological saline. In addition, the pharmaceutical composition or formulation may also include other carriers or non-toxic, non-therapeutic, non-immunogenic stabilizers and the like. Thus, the formulated Neurotoxin product can be present, in an aspect, in liquid or lyophilized form. In an aspect, it can be present together with glycerol, protein stabilizers (HSA) or non-protein stabilizers such as polyvinyl pyrrolidone (PVP), hyaluronic acid or free amino acids. In an aspect, suitable non-proteinaceous stabilizers are disclosed in WO 2005/007185 or WO 2006/020208. The formulated Neurotoxin product may be used for human or animal therapy of various diseases or disorders in a therapeutically effective dose or for cosmetic purposes.

In particular embodiments, the recombinant clostridial neurotoxin of the present invention or the pharmaceutical composition of the present invention is for use in the treatment of a disease or condition taken from the list of: wound healing, immobilisation for bone and tendon fracture treatment, post surgery immobilization, specifically in connection with haemorrhoidectomy, introduction of dental implants, or hip joint replacement (endoprothesis), epicondylitis, knee arthroplasty, ophthalmo logical surgery, acne, irritable bowel disease, vaginism, low back pain, or benign prostate dysplasia.

In yet another aspect, the present invention relates to the use of the composition of the present invention for cosmetic treatment.

In the context of the present invention, the term "cosmetic treatment" relates to uses in cosmetic or aesthetic applications, such as the treatment of wrinkles, crow's feet, glabella frown lines, reduction of the masseter muscle, reduction of the calves, removing of facial assymetries etc..

In another aspect, the present invention relates to a method for the generation of the recombinant clostridial neurotoxin of the present invention, comprising the step of obtaining a recombinant nucleic acid sequence encoding a recombinant single-chain precursor clostridial neurotoxin by the insertion of a nucleic acid sequence encoding said domain consisting of proline, valine and threonine residues into a nucleic acid sequence encoding a parental clostridial neurotoxin.

In the context of the present invention, the term "recombinant nucleic acid sequence" refers to a nucleic acid, which has been generated by joining genetic material from two different sources.

In the context of the present invention, the term "single-chain precursor clostridial neurotoxin" refers to a single-chain precursor for a disulfide-linked di-chain clostridial neurotoxin, comprising a functionally active clostridial neurotoxin light chain, a functionally active neurotoxin heavy chain, and a loop region linking the C-terminus of the light chain with the N-terminus of the heavy chain.

In the context of the present invention, the term "recombinant single-chain precursor clostridial neurotoxin" refers to a single-chain precursor clostridial neurotoxin comprising a heterologous domain, i.e. a domain from a species other than *Clostridium botulinum.*

In particular embodiments, the recombinant single-chain precursor clostridial neurotoxin comprises a protease cleavage site in said loop region.

Single-chain precursor clostridial neurotoxins have to be proteolytically cleaved to obtain the final biologically active clostridial neurotoxins. Proteolytic cleavage may either occur during heterologous expression by host cell enzymes, or by adding proteolytic enzymes to the raw protein material isolated after heterologous expression. Naturally occurring clostridial neurotoxins usually contain one or more cleavage signals for proteases which post-translationally cleave the single-chain precursor molecule, so that the final di- or multimeric complex can form. At present, clostridial neurotoxins are still predominantly produced by fermentation processes using appropriate Clostridium strains. During the fermentation process, the single-chain precursors are proteolytically cleaved by an unknown clostridial protease to obtain the biologically active di-chain clostridial neurotoxin. In cases, where the single-chain precursor molecule is the precursor of a protease, autocatalytic cleavage may occur. Alternatively, the protease can be a separate non-clostridial enzyme expressed in the same cell. WO 2006/076902 describes the proteolytic cleavage of a recombinant clostridial neurotoxin single-chain precursor at a heterologous recognition and cleavage site by incubation of the E. *coli* host cell lysate. The proteolytic cleavage is carried out by an unknown *E*. *coli* protease. In certain applications of recombinant expression, modified protease cleavage sites have been introduced recombinantly into the interchain region between the light and heavy chain of clostridial toxins, e.g. protease cleavage sites for human thrombin or non-human proteases (see WO 01/14570).

In particular embodiments, the protease cleavage site is a site that is cleaved by a protease selected from the list of: a protease selected from the list of: thrombin, trypsin, enterokinase, factor Xa, plant papain, insect papain, crustacean papain, enterokinase, human rhinovirus 3C protease, human enterovirus 3C protease, tobacco etch virus protease, Tobacco Vein Mottling Virus, subtilisin and caspase 3.

In a particular embodiment, the recombinant single-chain precursor clostridial neurotoxin further comprises a binding tag, particularly selected from the group comprising: glutathione-S-transferase (GST), maltose binding protein (MBP), a His-tag, a StrepTag, or a FLAG-tag.

In the context of the present invention, the term "parental clostridial neurotoxin" refers to an initial clostridial neurotoxin without a heterologous domain consisting of proline, valine and threonine residues, selected from a natural clostridial neurotoxin, a functional variant of a natural clostridial neurotoxin or a chimeric clostridial neurotoxin, wherein the clostridial neurotoxin light chain and heavy chain are from different clostridial neurotoxin serotypes.

In particular embodiments, the method for the generation of the recombinant clostridial neurotoxin of the present invention further comprises the step of heterologously expressing said recombinant nucleic acid sequence in a host cell, particularly in a bacterial host cell, more particularly in an *E*. *coli* host cell.

In certain embodiments, the E. *coli* cells are selected from *E*. *coli* XL1-Blue, Nova Blue, TOP10, XL10-Gold, BL21, and K12.

In particular embodiments, the method for the generation of the recombinant clostridial neurotoxin of the present invention additionally comprises at least one of the steps of (i) generating a disulfide-linked di-chain recombinant clostridial neurotoxin comprising said domain consisting of proline, valine and threonine residues by causing or allowing contacting of said recombinant single-chain precursor clostridial neurotoxin with an endoprotease and (ii) purification of said recombinant single-chain precursor clostridial neurotoxin or said disulfide-linked di-chain recombinant clostridial neurotoxin by chromatography.

In particular embodiments, the recombinant single-chain precursor clostridial neurotoxin, or the recombinant disulfide-linked di-chain clostridial neurotoxin, is purified after expression, or in the case of the recombinant disulfide-linked di-chain clostridial neurotoxin, after the cleavage reaction. In particular such embodiments, the protein is purified by chromatography, particularly by immunoaffinity chromatography, or by chromatography on an ion exchange matrix, a hydrophobic interaction matrix, or a multimodal chromatography matrix, particularly a strong ion exchange matrix, more particularly a strong cation exchange matrix.

In the context of the present invention, the term "causing ... contacting of said recombinant single-chain precursor clostridial neurotoxin ...with an endoprotease" refers to an active and/or direct step of bringing said neurotoxin and said endoprotease in contact, whereas the term "allowing contacting of a recombinant single-chain precursor clostridial neurotoxin ...with an endoprotease" refers to an indirect step of establishing conditions in such a way that said neurotoxin and said endoprotease are getting in contact to each other.

In the context of the present invention, the term "endoprotease" refers to a protease that breaks peptide bonds of non-terminal amino acids (i.e. within the polypeptide chain). As they do not attack terminal amino acids, endoproteases cannot break down peptides into monomers.

In particular embodiments, cleavage of the recombinant single-chain precursor clostridial neurotoxin is near-complete.

In the context of the present invention, the term "near-complete" is defined as more than about 95% cleavage, particularly more than about 97.5%, more particularly more than about 99% as determined by SDS-PAGE and subsequent Western Blot or reversed phase chromatography.

In particular embodiments, cleavage of the recombinant single-chain precursor clostridial neurotoxin occurs at a heterologous cleavage signal located in the loop region of the recombinant precursor clostridial neurotoxin.

In particular embodiments, the cleavage reaction is performed with crude host cell lysates containing said single-chain precursor protein.

In other particular embodiments, the single-chain precursor protein is purified or partially purified, particularly by a first chromatographic enrichment step, prior to the cleavage reaction.

In the context of the present invention, the term "purified" relates to more than about 90% purity. In the context of the present invention, the term "partially purified" relates to purity of less than about 90% and an enrichment of more than about two fold.

In another aspect, the present invention relates to a recombinant single-chain clostridial neurotoxin, which is a precursor for the recombinant clostridial neurotoxin of the present invention Thus, in such aspect, the present invention relates to a recombinant single-chain precursor clostridial neurotoxin comprising a domain consisting of proline, valine and threonine residues.

In particular embodiments, said domain consisting of proline, valine and threonine residues comprises an amino acid sequence consisting of at least 50 amino acid residues, particularly between 50 and 3000 amino acid residues, more particularly between 60 and 500 amino acid residues, more particularly between 70 and 260 amino acid residues, more particularly between 80 and 240 amino acid residues, more particularly between 90 and 220 amino acid residues, particularly 100 amino acid residues, 150 amino acid residues, or 200 amino acid residues.

In particular embodiments, said domain comprises a plurality of amino acid repeats, wherein said repeat consist of Pro, Val and Thr residues and wherein no more than 6 consecutive amino acid residues are identical.

In particular embodiments, the proline residues comprised in said domain constitute more than 4% and less than 40% of the amino acids of said domain.

In particular embodiments, said domain comprises at least the following amino acid sequence VTPVVPVPVTPVVPVPTVPV (SEQ ID NO: 1) or circular permuted versions or at least one multimer of the sequence as a whole or parts of the sequence, particularly (VTPVVPVPVTPVVPVPTVPV)ₙ, with n being an integer selected from 3 to 25, in particular wherein n is 5.

In particular embodiments, said domain is inserted at (i) the N-terminus of the light chain of said recombinant clostridial neurotoxin; (ii) the C-terminus of the light chain of said recombinant clostridial neurotoxin; (i) the N-terminus of the heavy chain of said recombinant clostridial neurotoxin; or (ii) the C-terminus of the heavy chain of said recombinant clostridial neurotoxin.

In a particular preferred embodiment of the invention the recombinant clostridial neurotoxin, comprises a domain consisting of (VTPVVPVPVTPVVPVPTVPV)₅ at the N-terminus of the light chain of said recombinant clostridial neurotoxin.

In particular embodiments, the sequence of said clostridial neurotoxin is selected from the sequence of (i) a *Clostridium botulinum* neurotoxin serotype A, B, C, D, E, F, and G, particularly *Clostridium botulinum* neurotoxin serotype A, C and E, more particularly *Clostridium botulinum* neurotoxin serotype A, or (ii) from the sequence of a functional variant of a *Clostridium botulinum* neurotoxin of (i), or (iii) from the sequence of a chimeric *Clostridium botulinum* neurotoxin, wherein the clostridial neurotoxin light chain and heavy chain are from different clostridial neurotoxin serotypes.

In particular embodiments of the present invention, said recombinant single-chain clostridial neurotoxin has the amino acid sequence as found in in SEQ ID NO: 2 (see Table 1).

In another aspect, the present invention relates to a nucleic acid sequence encoding the recombinant single-chain clostridial neurotoxin of the present invention, particularly a nucleic acid sequence as found in SEQ ID NO: 3 (see Table 1).

In another aspect, the present invention relates to a method for obtaining the nucleic acid sequence of the present invention, comprising the step of inserting a nucleic acid sequence encoding said domain into a nucleic acid sequence encoding a parental clostridial neurotoxin.

In another aspect, the present invention relates to a vector comprising the nucleic acid sequence of the present invention, or the nucleic acid sequence obtainable by the method of the present invention.

In another aspect, the present invention relates to a recombinant host cell comprising the nucleic acid sequence of the present invention, the nucleic acid sequence obtainable by the method of the present invention, or the vector of the present invention.

In certain embodiments, the recombinant host cells are selected from *E*. *coli* XL1-Blue, Nova Blue, TOP10, XL10-Gold, BL21, and K12.

In another aspect, the present invention relates to a method for producing the recombinant single-chain precursor clostridial neurotoxin of the present invention, comprising the step of expressing the nucleic acid sequence of the present invention, or the nucleic acid sequence obtainable by the method of the present invention, or the vector of the present invention in a recombinant host cell, or cultivating the recombinant host cell of the present invention under conditions that result in the expression of said nucleic acid sequence.

### EXAMPLES

### Example 1: Generation and Purification of a PVT Botulinum Toxin Type A (PVT-100-rBoNT/A)

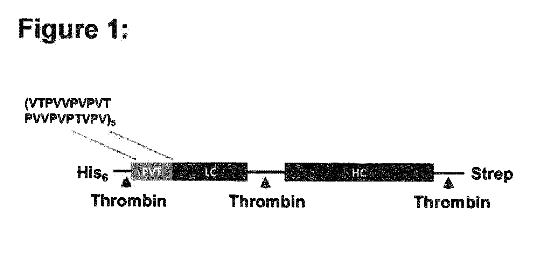

The "PVT" module comprising 100 amino acid residues built from the amino acids proline, valine and threonine was synthetically produced and after digestion with Ndel and Swal inserted at the N-terminus of recombinant BoNT/A (PVT-100-rBoNT/A) (Figure 1). The correct cloning was verified by sequencing.

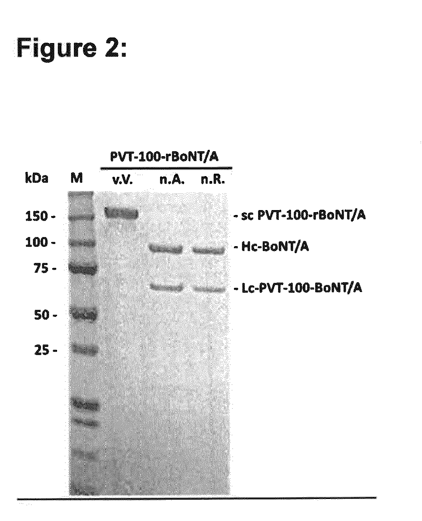

Expression was performed in expression strain *E*. *coli* BI21. Purification was done using a combination of his affinity, ion exchange and size exclusion chromatography, followed by activation using thrombin. Figure 2 summarizes the results of purification and activation.

### Example 2: Duration of Effect of PVT-100-rBoNT/A in a "Mouse Running Assay"

Three different dosages of PVT-100-rBoNT/A, i.e. 5,0 pg, 7,0 pg and 9,0 pg were injected into the M. gastrocnemius of each mice in comparison to standard Xeomin^{®}. The mice had been trained in a treadmill. The daily running distance in the treadmill was measured over 20 days. The paralysis caused by the toxins was plotted as percentage of the running distance on the day before the injection, which was set as 100%, against the time (see **Figure 3****).**

The injection of PVT-100-rBoNT/A resulted in a maximum paralysis after 3 days, for the control group treated with Xeomin maximum paralysis was observed after 3 days as well. Because the recovery is dose dependant equipotent doses must be compared. During the recovery phase after maximum paralysis the running distance of the control group (BoNT/A1) reached a value of 40% of the starting value after 9.3 days, whereas the group treated with the equipotent dose of PVT-100-rBoNT/A (7 pg) reached that value at 7.3 days. Thus, the duration of effective paralysis was markedly shortened.

**Table 1: Sequences**

| |
|---|
| SEQ ID NO 1: |
| VTPVVPVPVTPVVPVPTVPV |
| |
| SEQ ID NO 2: |
| PVT-100-BoNT/A |
| |
| MGSSHHHHHHGSLVPRGTPVVPVPVTPVVPVPTVPVVTPVVPVPVTPVVPVPTVPVVTPV VPVPVTPVVPVPTVPVVTPVVPVPVTPVVPVPTVPVVTPVVPVPVTPVVPVPTVPVVPFV NKQFNYKDPVNGVDIAYIKIPNAGQMQPVKAFKIHNKIWVIPERDTFTNPEEGDLNPPPE AKQVPVSYYDSTYLSTDNEKDNYLKGVTKLFERIYSTDLGRMLLTSIVRGIPFWGGSTID TELKVIDTNCINVIQPDGSYRSEELNLVIIGPSADIIQFECKSFGHEVLNLTRNGYGSTQ YIRFSPDFTFGFEESLEVDTNPLLGAGKFATDPAVTLAHELIHAGHRLYGIAINPNRVFK VNTNAYYEMSGLEVSFEELRTFGGHDAKFIDSLQENEFRLYYYNKFKDIASTLNKAKSIV GTTASLQYMKNVFKEKYLLSEDTSGKFSVDKLKFDKLYKMLTEIYTEDNFVKFFKVLNRK TYLNFDKAVFKINIVPKVNYTIYDGFNLRNTNLAANFNGQNTEINNMNFTKLKNFTGLFE FYKLLCVRGIITSKAGAGKSLVPRGSAGAGALNDLCIKVNNWDLFFSPSEDNFTNDLNKG EEITSDTNIEAAEENISLDLIQQYYLTFNFDNEPENISIENLSSDIIGQLELMPNIERFP |
| NGKKYELDKYTMFHYLRAQEFEHGKSRIALTNSVNEALLNPSRVYTFFSSDYVKKVNKAT EAAMFLGWVEQLVYDFTDETSEVSTTDKIADITIIIPYIGPALNIGNMLYKDDFVGALIF SGAVILLEFIPEIAIPVLGTFALVSYIANKVLTVQTIDNALSKRNEKWDEVYKYIVTNWL AKVNTQIDLIRKKMKEALENQAEATKAIINYQYNQYTEEEKNNINFNIDDLSSKLNESIN KAMININKFLNQCSVSYLMNSMIPYGVKRLEDFDASLKDALLKYIYDNRGTLIGQVDRLK DKVNNTLSTDIPFQLSKYVDNQRLLSTFTEYIKNIINTSILNLRYESNHLIDLSRYASKI NIGSKVNFDPIDKNQIQLFNLESSKIEVILKNAIVYNSMYENFSTSFWIRIPKYFNSISL NNEYTIINCMENNSGWKVSLNYGEIIWTLQDTQEIKQRVVFKYSQMINISDYINRWIFVT ITNNRLNNSKIYINGRLIDQKPISNLGNIHASNNIMFKLDGCRDTHRYIWIKYFNLFDKE LNEKEIKDLYDNQSNSGILKDFWGDYLQYDKPYYMLNLYDPNKYVDVNNVGIRGYMYLKG PRGSVMTTNIYLNSSLYRGTKFIIKKYASGNKDNIVRNNDRVYINVVVKNKEYRLATNAS QAGVEKILSALEIPDVGNLSQVVVMKSKNDQGITNKCKMNLQDNNGNDIGFIGFHQFNNI AKLVASNWYNRQIERSSRTLGCSWEFIPVDDGWGERPLGDLVPRGSANSSSVDKLWSHPQ FEK |
| |
| SEQ ID NO 3: |
| PVT-100-BoNT/A (4332 bp) |
| ATGGGTAGCAGCCATCATCATCACCATCATGGTAGCCTGGTTCCGCGTGGTACACCGGTT GTTCCGGTTCCGGTTACACCGGTGGTGCCTGTGCCGACCGTTCCGGTTGTTACCCCTGTT GTGCCGGTGCCGGTGACCCCAGTTGTTCCAGTGCCGACAGTGCCGGTAGTGACTCCGGTG GTTCCAGTTCCTGTGACCCCTGTAGTACCGGTTCCGACCGTGCCTGTTGTGACGCCAGTA GTGCCTGTACCGGTGACACCTGTCGTGCCAGTTCCTACAGTTCCAGTTGTGACACCAGTC GTACCGGTGCCTGTTACGCCTGTCGTTCCTGTTCCAACTGTGCCGGTTGTGCCATTTGTG AACAAGCAGTTTAACTATAAGGACCCGGTGAACGGTGTGGATATCGCGTATATCAAAATC CCGAATGCGGGCCAGATGCAACCAGTCAAGGCGTTCAAGATTCATAACAAGATTTGGGTT ATTCCGGAACGTGATACCTTCACCAATCCGGAAGAAGGCGATTTAAATCCGCCGCCAGAA |
| GCCAAACAAGTGCCGGTGAGCTACTATGATAGCACGTATCTTAGCACCGATAATGAAAAA GACAATTACCTGAAGGGCGTGACCAAGTTGTTCGAGCGCATCTACAGTACCGACTTAGGC CGCATGTTGTTGACGAGCATCGTTCGCGGTATCCCGTTCTGGGGCGGCTCGACCATTGAT ACCGAGTTGAAAGTCATTGACACGAACTGTATCAATGTTATCCAACCGGACGGCAGTTAT CGCAGCGAGGAGTTAAATTTGGTCATCATCGGTCCAAGCGCAGATATTATTCAGTTCGAA TGCAAGAGCTTCGGCCATGAGGTCTTGAATTTGACGCGCAACGGTTACGGCAGCACCCAA TACATCCGCTTTAGCCCGGATTTCACCTTTGGCTTCGAGGAGAGCTTGGAGGTGGACACC AACCCGCTGTTAGGTGCCGGCAAATTCGCAACCGACCCGGCAGTGACGTTGGCGCACGAA TTGATTCATGCGGGTCACCGCTTATACGGTATCGCGATCAATCCGAATCGCGTCTTTAAA GTCAATACCAACGCGTACTACGAAATGAGCGGCTTAGAGGTTAGCTTTGAAGAATTACGC ACCTTCGGTGGCCACGACGCCAAGTTCATCGACAGCCTGCAGGAAAATGAGTTCCGCTTG TACTATTACAATAAATTCAAGGACATCGCGAGCACCTTAAATAAAGCAAAGAGCATTGTG GGCACCACCGCAAGCTTGCAGTACATGAAGAACGTATTTAAGGAAAAATATTTGTTGTCG GAGGATACCAGCGGGAAATTCAGCGTCGATAAGCTGAAATTCGACAAATTGTATAAAATG CTGACCGAGATTTACACCGAGGATAACTTCGTCAAGTTTTTTAAGGTGTTAAATCGTAAG ACCTATTTAAACTTTGATAAAGCGGTGTTTAAAATTAATATCGTGCCGAAGGTGAATTAC ACCATCTACGATGGTTTCAATTTACGCAACACGAATCTGGCGGCGAATTTTAATGGCCAA AACACCGAAATTAACAACATGAACTTTACGAAGTTAAAGAATTTCACGGGCTTATTCGAA TTCTACAAGTTATTATGCGTGCGCGGCATCATTACCAGCAAGGCAGGTGCGGGCAAGTCC TTGGTTCCGCGTGGCAGCGCCGGCGCCGGCGCGCTCAATGATCTGTGTATTAAAGTCAAT AACTGGGACCTGTTCTTCAGCCCGAGCGAGGATAACTTTACCAACGACTTAAACAAAGGC GAGGAGATCACGAGCGATACGAACATCGAGGCGGCGGAGGAAAATATTAGCCTGGACCTC ATTCAGCAGTACTATCTGACGTTCAATTTTGACAATGAGCCGGAGAACATCAGCATTGAA AATCTCAGCAGCGACATCATCGGTCAGTTGGAACTGATGCCGAACATTGAACGCTTTCCG AACGGCAAAAAATATGAACTGGACAAGTATACCATGTTCCATTACTTACGCGCACAGGAA |
| TTTGAGCACGGCAAGAGCCGCATTGCGCTGACCAATAGCGTTAACGAGGCCTTGTTAAAT CCGAGCCGTGTCTACACGTTCTTCAGCAGCGATTATGTCAAAAAGTGAACAAGGCGACC GAAGCCGCGATGTTTTTGGGCTGGGTCGAGCAATTGGTTTACGATTTTACCGACGAAACC AGCGAGGTGAGCACGACCGACAAAATTGCAGATATCACCATCATCATTCCGTACATCGGT CCGGCGCTCAATATCGGCAATATGTTATACAAGGACGACTTTGTGGGCGCGCTGATCTTT AGCGGCGCGGTTATCTTATTAGAATTCATCCCGGAGATCGCAATCCCGGTCTTGGGCACC TTTGCGTTGGTGAGCTATATCGCGAATAAAGTGCTCACGGTCCAAACCATCGATAACGCG CTCAGCAAGCGTAATGAGAAATGGGACGAGGTTTATAAGTATATCGTGACCAACTGGTTA GCAAAAGTCAATACGCAGATCGATCTCATCCGCAAAAAAATGAAAGAAGCCTTGGAAAAT CAAGCGGAGGCAACCAAAGCCATCATTAATTACCAGTATAACCAATATACCGAAGAAGAA AAAAACAATATCAACTTCAATATCGATGATTTGAGCAGCAAACTGAACGAGAGCATTAAC AAAGCGATGATTAACATCAACAAGTTCTTGAATCAATGCAGCGTGAGCTATCTCATGAAC AGCATGATCCCGTATGGCGTCAAACGCTTGGAAGATTTTGACGCCAGCCTGAAAGATGCG CTCCTCAAGTATATTTATGACAACCGCGGCACCCTCATTGGCCAGGTGGACCGCTTGAAG GATAAAGTGAACAATACGCTCAGCACGGATATCCCGTTCCAGCTGAGCAAGTACGTCGAC AACCAGCGCTTACTGAGCACCTTTACCGAGTATATCAAGAACATCATTAATACCAGCATC CTCAACTTGCGCTATGAGAGCAATCACCTGATCGACCTCAGCCGCTACGCCAGCAAGATC AACATCGGCAGCAAGGTCAATTTCGACCCGATCGATAAGAATCAGATCCAATTGTTTAAC CTGGAAAGCAGCAAGATCGAGGTTATCTTGAAGAACGCGATTGTGTACAACAGCATGTAC GAGAACTTTAGCACGAGCTTCTGGATTCGTATCCCGAAGTATTTCAATAGCATTAGCCTG AATAACGAATATACCATTATCAACTGCATGGAAAATAATAGCGGCTGGAAGGTGAGCTTA AATTACGGCGAGATCATTTGGACCTTACAGGATACCCAAGAAATCAAACAGCGCGTCGTC TTTAAGTATAGCCAGATGATCAACATCAGCGATTACATCAACCGCTGGATCTTCGTGACC ATCACCAATAATCGCTTGAATAATAGCAAGATTTACATCAATGGTCGCTTGATTGATCAA AAACCGATCAGCAATCTCGGTAATATCCATGCCAGCAATAACATCATGTTTAAGTTAGAC |
| GGTTGCCGCGATACCCACCGCTATATCTGGATCAAGTATTTTAACTTATTTGATAAGGAA CTCAACGAAAAGGAAATTAAAGACTTATATGACAATCAGAGCAATAGCGGCATCCTGAAG GATTTCTGGGGCGACTACCTGCAGTACGATAAGCCGTACTATATGTTGAACTTGTATGAC CCGAACAAATATGTCGATGTGAACAATGTGGGTATTCGTGGCTATATGTACTTAAAGGGC CCGCGTGGTAGCGTGATGACCACGAATATTTACTTAAACAGCAGCTTATACCGCGGCACG AAGTTTATTATCAAGAAGTATGCCAGCGGCAACAAGGACAATATCGTCCGCAACAACGAC CGTGTGTATATTAACGTGGTGGTGAAGAATAAAGAGTACCGCTTGGCCACGAATGCGAGC CAGGCGGGCGTGGAAAAAATCTTGAGCGCGTTGGAGATCCCGGACGTCGGCAACCTCAGC CAGGTTGTGGTGATGAAGTCTAAAAACGACCAGGGCATCACGAACAAGTGCAAAATGAAT TTGCAAGATAACAACGGCAACGACATCGGCTTTATTGGTTTTCACCAGTTCAATAACATC GCCAAACTCGTGGCCAGCAATTGGTATAACCGCCAAATTGAACGCAGCAGCCGCACGCTC GGCTGTAGCTGGGAGTTCATCCCGGTGGACGATGGCTGGGGCGAGCGCCCGCTCGGAGAT CTGGTGCCACGCGGTTCCGCGAATTCGAGCTCCGTCGACAAGCTTTGGAGCCACCCGCAG TTCGAAAAATAA |

## Claims

1. A recombinant clostridial neurotoxin or a functional variant thereof comprising a domain wherein said domain comprises an amino acid sequence consisting of at least 50 amino acid residues, wherein said domain consists of proline, valine and threonine residues, or wherein said domain comprises a plurality of amino acid repeats, wherein said repeat consist of Pro, Val and Thr residues and wherein no more than six consecutive amino acid residues are identical.

2. The recombinant clostridial neurotoxin of claim 1, wherein said domain comprises at least the following amino acid sequence VTPVVPVPVTPVVPVPTVPV (SEQ ID NO: 1) or circular permuted versions or multimers of the sequence as a whole or parts of the sequence, particularly (VTPVVPVPVTPVVPVPTVPV)ₙ, with n being an integer selected from 3 to 25, in particular wherein n is 5.

3. The recombinant clostridial neurotoxin of claim 1 or 2, wherein said domain is inserted at (i) the N-terminus of the light chain of said recombinant clostridial neurotoxin; (ii) the C-terminus of the light chain of said recombinant clostridial neurotoxin; (iii) the N-terminus of the heavy chain of said recombinant clostridial neurotoxin; or (iv) the C-terminus of the heavy chain of said recombinant clostridial neurotoxin.

4. The recombinant clostridial neurotoxin of any one of claims 1 to 3, wherein the sequence of said clostridial neurotoxin is selected from the sequence of (i) a *Clostridium botulinum* neurotoxin serotype A, B, C, D, E, F, and G, or (ii) from the sequence of a functional variant of a *Clostridium botulinum* neurotoxin of (i), or (iii) from the sequence of a chimeric *Clostridium botulinum* neurotoxin, wherein the clostridial neurotoxin light chain and heavy chain are from different clostridial neurotoxin serotypes.

5. The recombinant clostridial neurotoxin of claim 4, wherein said recombinant clostridial neurotoxin has a light chain and a heavy chain comprised in the amino acid sequence as found in SEQ ID NO: 2.

6. The recombinant clostridial neurotoxin of any one of claims 1 to 5, wherein said recombinant clostridial neurotoxin shows decreased duration of effect relative to an identical clostridial neurotoxin without said domain.

7. The recombinant clostridial neurotoxin of any one of claims 1 to 6 for the use in the treatment of a disease requiring improved chemodenervation, wherein the recombinant clostridial neurotoxin causes shorter lasting denervation relative to an identical clostridial neurotoxin without said domain.

8. A pharmaceutical composition comprising the recombinant clostridial neurotoxin of any one of claims 1 to 7.

9. Use of the recombinant clostridial neurotoxin of any one of claims 1 to 7 for cosmetic treatment.

10. A method for the generation of a recombinant clostridial neurotoxin according to any one of claims 1 to 7, comprising the step of obtaining a recombinant nucleic acid sequence encoding a recombinant single-chain precursor clostridial neurotoxin by the insertion of a nucleic acid sequence encoding said domain into a nucleic acid sequence encoding a parental clostridial neurotoxin, or wherein said method further comprises the step of heterologously expressing said recombinant nucleic acid sequence in a host cell, particularly in a bacterial host cell, more particularly in an *E*. *coli* host cell.

11. A recombinant single-chain clostridial neurotoxin, which is a precursor for the recombinant clostridial neurotoxin of any one of claims 1 to 7, e. g. wherein the sequence of said clostridial neurotoxin is selected from the sequence of (i) a Clostridium botulinum neurotoxin serotype A, B, C, D, E, F, and G, or (ii) from the sequence of a functional variant of a Clostridium botulinum neurotoxin of (i), or (iii) from the sequence of a chimeric Clostridium botulinum neurotoxin, wherein the clostridial neurotoxin light chain and heavy chain are from different clostridial neurotoxin serotypes.

12. A nucleic acid sequence encoding the recombinant single-chain clostridial neurotoxin of claim 11, particularly a nucleic acid sequence as found in SEQ ID NO: 3.

13. A method for obtaining the nucleic acid sequence of claim 12, comprising the step of inserting a nucleic acid sequence encoding said domain into a nucleic acid sequence encoding a parental clostridial neurotoxin.

14. A vector comprising the nucleic acid sequence of claim 12, or the nucleic acid sequence obtainable by the method of claim 13.

15. A recombinant host cell comprising the nucleic acid sequence of claim 12, the nucleic acid sequence obtainable by the method of claim 13, or the vector of claim 14.


SEQUENCE LISTING

<110> Merz Pharma GmbH & Co. KGaA

<120> NOVEL RECOMBINANT CLOSTRIDIAL NEUROTOXINS WITH DECREASED DURATION OF
EFFECT

<130> 4215-000

<160> 3

<170> BiSSAP 1.3.5

<210> 1
<211> 20
<212> PRT
<213> Artificial Sequence

<223> "Artificial Sequence"

<220>
<223> Artificial Sequence

<400> 1
Val Thr Pro Val Val Pro Val Pro Val Thr Pro Val Val Pro Val Pro
1               5                   10                  15
Thr Val Pro Val
            20

<210> 2
<211> 1443
<212> PRT
<213> Artificial Sequence

<223> "Artificial Sequence"

<220>
<223> Artificial Sequence

<400> 2
Met Gly Ser Ser His His His His His His Gly Ser Leu Val Pro Arg
1               5                   10                  15
Gly Thr Pro Val Val Pro Val Pro Val Thr Pro Val Val Pro Val Pro
            20                  25                  30
Thr Val Pro Val Val Thr Pro Val Val Pro Val Pro Val Thr Pro Val
        35                  40                  45
Val Pro Val Pro Thr Val Pro Val Val Thr Pro Val Val Pro Val Pro
    50                  55                  60
Val Thr Pro Val Val Pro Val Pro Thr Val Pro Val Val Thr Pro Val
65                  70                  75                  80
Val Pro Val Pro Val Thr Pro Val Val Pro Val Pro Thr Val Pro Val
                85                  90                  95
Val Thr Pro Val Val Pro Val Pro Val Thr Pro Val Val Pro Val Pro
            100                 105                 110
Thr Val Pro Val Val Pro Phe Val Asn Lys Gln Phe Asn Tyr Lys Asp
        115                 120                 125
Pro Val Asn Gly Val Asp Ile Ala Tyr Ile Lys Ile Pro Asn Ala Gly
    130                 135                 140
Gln Met Gln Pro Val Lys Ala Phe Lys Ile His Asn Lys Ile Trp Val
145                 150                 155                 160
Ile Pro Glu Arg Asp Thr Phe Thr Asn Pro Glu Glu Gly Asp Leu Asn
                165                 170                 175
Pro Pro Pro Glu Ala Lys Gln Val Pro Val Ser Tyr Tyr Asp Ser Thr

            180                 185                 190
Tyr Leu Ser Thr Asp Asn Glu Lys Asp Asn Tyr Leu Lys Gly Val Thr
        195                 200                 205
Lys Leu Phe Glu Arg Ile Tyr Ser Thr Asp Leu Gly Arg Met Leu Leu
    210                 215                 220
Thr Ser Ile Val Arg Gly Ile Pro Phe Trp Gly Gly Ser Thr Ile Asp
225                 230                 235                 240
Thr Glu Leu Lys Val Ile Asp Thr Asn Cys Ile Asn Val Ile Gln Pro
                245                 250                 255
Asp Gly Ser Tyr Arg Ser Glu Glu Leu Asn Leu Val Ile Ile Gly Pro
            260                 265                 270
Ser Ala Asp Ile Ile Gln Phe Glu Cys Lys Ser Phe Gly His Glu Val
        275                 280                 285
Leu Asn Leu Thr Arg Asn Gly Tyr Gly Ser Thr Gln Tyr Ile Arg Phe
    290                 295                 300
Ser Pro Asp Phe Thr Phe Gly Phe Glu Glu Ser Leu Glu Val Asp Thr
305                 310                 315                 320
Asn Pro Leu Leu Gly Ala Gly Lys Phe Ala Thr Asp Pro Ala Val Thr
                325                 330                 335
Leu Ala His Glu Leu Ile His Ala Gly His Arg Leu Tyr Gly Ile Ala
            340                 345                 350
Ile Asn Pro Asn Arg Val Phe Lys Val Asn Thr Asn Ala Tyr Tyr Glu
        355                 360                 365
Met Ser Gly Leu Glu Val Ser Phe Glu Glu Leu Arg Thr Phe Gly Gly
    370                 375                 380
His Asp Ala Lys Phe Ile Asp Ser Leu Gln Glu Asn Glu Phe Arg Leu
385                 390                 395                 400
Tyr Tyr Tyr Asn Lys Phe Lys Asp Ile Ala Ser Thr Leu Asn Lys Ala
                405                 410                 415
Lys Ser Ile Val Gly Thr Thr Ala Ser Leu Gln Tyr Met Lys Asn Val
            420                 425                 430
Phe Lys Glu Lys Tyr Leu Leu Ser Glu Asp Thr Ser Gly Lys Phe Ser
        435                 440                 445
Val Asp Lys Leu Lys Phe Asp Lys Leu Tyr Lys Met Leu Thr Glu Ile
    450                 455                 460
Tyr Thr Glu Asp Asn Phe Val Lys Phe Phe Lys Val Leu Asn Arg Lys
465                 470                 475                 480
Thr Tyr Leu Asn Phe Asp Lys Ala Val Phe Lys Ile Asn Ile Val Pro
                485                 490                 495
Lys Val Asn Tyr Thr Ile Tyr Asp Gly Phe Asn Leu Arg Asn Thr Asn
            500                 505                 510
Leu Ala Ala Asn Phe Asn Gly Gln Asn Thr Glu Ile Asn Asn Met Asn
        515                 520                 525
Phe Thr Lys Leu Lys Asn Phe Thr Gly Leu Phe Glu Phe Tyr Lys Leu
    530                 535                 540
Leu Cys Val Arg Gly Ile Ile Thr Ser Lys Ala Gly Ala Gly Lys Ser
545                 550                 555                 560
Leu Val Pro Arg Gly Ser Ala Gly Ala Gly Ala Leu Asn Asp Leu Cys
                565                 570                 575
Ile Lys Val Asn Asn Trp Asp Leu Phe Phe Ser Pro Ser Glu Asp Asn
            580                 585                 590
Phe Thr Asn Asp Leu Asn Lys Gly Glu Glu Ile Thr Ser Asp Thr Asn
        595                 600                 605
Ile Glu Ala Ala Glu Glu Asn Ile Ser Leu Asp Leu Ile Gln Gln Tyr
    610                 615                 620
Tyr Leu Thr Phe Asn Phe Asp Asn Glu Pro Glu Asn Ile Ser Ile Glu
625                 630                 635                 640
Asn Leu Ser Ser Asp Ile Ile Gly Gln Leu Glu Leu Met Pro Asn Ile
                645                 650                 655
Glu Arg Phe Pro Asn Gly Lys Lys Tyr Glu Leu Asp Lys Tyr Thr Met
            660                 665                 670
Phe His Tyr Leu Arg Ala Gln Glu Phe Glu His Gly Lys Ser Arg Ile
        675                 680                 685

Ala Leu Thr Asn Ser Val Asn Glu Ala Leu Leu Asn Pro Ser Arg Val
    690                 695                 700
Tyr Thr Phe Phe Ser Ser Asp Tyr Val Lys Lys Val Asn Lys Ala Thr
705                 710                 715                 720
Glu Ala Ala Met Phe Leu Gly Trp Val Glu Gln Leu Val Tyr Asp Phe
                725                 730                 735
Thr Asp Glu Thr Ser Glu Val Ser Thr Thr Asp Lys Ile Ala Asp Ile
            740                 745                 750
Thr Ile Ile Ile Pro Tyr Ile Gly Pro Ala Leu Asn Ile Gly Asn Met
        755                 760                 765
Leu Tyr Lys Asp Asp Phe Val Gly Ala Leu Ile Phe Ser Gly Ala Val
    770                 775                 780
Ile Leu Leu Glu Phe Ile Pro Glu Ile Ala Ile Pro Val Leu Gly Thr
785                 790                 795                 800
Phe Ala Leu Val Ser Tyr Ile Ala Asn Lys Val Leu Thr Val Gln Thr
                805                 810                 815
Ile Asp Asn Ala Leu Ser Lys Arg Asn Glu Lys Trp Asp Glu Val Tyr
            820                 825                 830
Lys Tyr Ile Val Thr Asn Trp Leu Ala Lys Val Asn Thr Gln Ile Asp
        835                 840                 845
Leu Ile Arg Lys Lys Met Lys Glu Ala Leu Glu Asn Gln Ala Glu Ala
    850                 855                 860
Thr Lys Ala Ile Ile Asn Tyr Gln Tyr Asn Gln Tyr Thr Glu Glu Glu
865                 870                 875                 880
Lys Asn Asn Ile Asn Phe Asn Ile Asp Asp Leu Ser Ser Lys Leu Asn
                885                 890                 895
Glu Ser Ile Asn Lys Ala Met Ile Asn Ile Asn Lys Phe Leu Asn Gln
            900                 905                 910
Cys Ser Val Ser Tyr Leu Met Asn Ser Met Ile Pro Tyr Gly Val Lys
        915                 920                 925
Arg Leu Glu Asp Phe Asp Ala Ser Leu Lys Asp Ala Leu Leu Lys Tyr
    930                 935                 940
Ile Tyr Asp Asn Arg Gly Thr Leu Ile Gly Gln Val Asp Arg Leu Lys
945                 950                 955                 960
Asp Lys Val Asn Asn Thr Leu Ser Thr Asp Ile Pro Phe Gln Leu Ser
                965                 970                 975
Lys Tyr Val Asp Asn Gln Arg Leu Leu Ser Thr Phe Thr Glu Tyr Ile
            980                 985                 990
Lys Asn Ile Ile Asn Thr Ser Ile Leu Asn Leu Arg Tyr Glu Ser Asn
        995                 1000                1005
His Leu Ile Asp Leu Ser Arg Tyr Ala Ser Lys Ile Asn Ile Gly Ser
    1010                1015                1020
Lys Val Asn Phe Asp Pro Ile Asp Lys Asn Gln Ile Gln Leu Phe Asn
1025                1030                1035                1040
Leu Glu Ser Ser Lys Ile Glu Val Ile Leu Lys Asn Ala Ile Val Tyr
                1045                1050                1055
Asn Ser Met Tyr Glu Asn Phe Ser Thr Ser Phe Trp Ile Arg Ile Pro
            1060                1065                1070
Lys Tyr Phe Asn Ser Ile Ser Leu Asn Asn Glu Tyr Thr Ile Ile Asn
        1075                1080                1085
Cys Met Glu Asn Asn Ser Gly Trp Lys Val Ser Leu Asn Tyr Gly Glu
    1090                1095                1100
Ile Ile Trp Thr Leu Gln Asp Thr Gln Glu Ile Lys Gln Arg Val Val
1105                1110                1115                1120
Phe Lys Tyr Ser Gln Met Ile Asn Ile Ser Asp Tyr Ile Asn Arg Trp
                1125                1130                1135
Ile Phe Val Thr Ile Thr Asn Asn Arg Leu Asn Asn Ser Lys Ile Tyr
            1140                1145                1150
Ile Asn Gly Arg Leu Ile Asp Gln Lys Pro Ile Ser Asn Leu Gly Asn
        1155                1160                1165
Ile His Ala Ser Asn Asn Ile Met Phe Lys Leu Asp Gly Cys Arg Asp
    1170                1175                1180
Thr His Arg Tyr Ile Trp Ile Lys Tyr Phe Asn Leu Phe Asp Lys Glu

1185                1190                1195                1200
Leu Asn Glu Lys Glu Ile Lys Asp Leu Tyr Asp Asn Gln Ser Asn Ser
                1205                1210                1215
Gly Ile Leu Lys Asp Phe Trp Gly Asp Tyr Leu Gln Tyr Asp Lys Pro
            1220                1225                1230
Tyr Tyr Met Leu Asn Leu Tyr Asp Pro Asn Lys Tyr Val Asp Val Asn
        1235                1240                1245
Asn Val Gly Ile Arg Gly Tyr Met Tyr Leu Lys Gly Pro Arg Gly Ser
    1250                1255                1260
Val Met Thr Thr Asn Ile Tyr Leu Asn Ser Ser Leu Tyr Arg Gly Thr
1265                1270                1275                1280
Lys Phe Ile Ile Lys Lys Tyr Ala Ser Gly Asn Lys Asp Asn Ile Val
                1285                1290                1295
Arg Asn Asn Asp Arg Val Tyr Ile Asn Val Val Val Lys Asn Lys Glu
            1300                1305                1310
Tyr Arg Leu Ala Thr Asn Ala Ser Gln Ala Gly Val Glu Lys Ile Leu
        1315                1320                1325
Ser Ala Leu Glu Ile Pro Asp Val Gly Asn Leu Ser Gln Val Val Val
    1330                1335                1340
Met Lys Ser Lys Asn Asp Gln Gly Ile Thr Asn Lys Cys Lys Met Asn
1345                1350                1355                1360
Leu Gln Asp Asn Asn Gly Asn Asp Ile Gly Phe Ile Gly Phe His Gln
                1365                1370                1375
Phe Asn Asn Ile Ala Lys Leu Val Ala Ser Asn Trp Tyr Asn Arg Gln
            1380                1385                1390
Ile Glu Arg Ser Ser Arg Thr Leu Gly Cys Ser Trp Glu Phe Ile Pro
        1395                1400                1405
Val Asp Asp Gly Trp Gly Glu Arg Pro Leu Gly Asp Leu Val Pro Arg
    1410                1415                1420
Gly Ser Ala Asn Ser Ser Ser Val Asp Lys Leu Trp Ser His Pro Gln
1425                1430                1435                1440
Phe Glu Lys


<210> 3
<211> 4332
<212> DNA
<213> Artificial Sequence

<223> "Artificial Sequence"

<220>
<223> Artificial Sequence

<400> 3
atgggtagca gccatcatca tcaccatcat ggtagcctgg ttccgcgtgg tacaccggtt      60

gttccggttc cggttacacc ggtggtgcct gtgccgaccg ttccggttgt tacccctgtt     120

gtgccggtgc cggtgacccc agttgttcca gtgccgacag tgccggtagt gactccggtg     180

gttccagttc ctgtgacccc tgtagtaccg gttccgaccg tgcctgttgt gacgccagta     240

gtgcctgtac cggtgacacc tgtcgtgcca gttcctacag ttccagttgt gacaccagtc     300

gtaccggtgc ctgttacgcc tgtcgttcct gttccaactg tgccggttgt gccatttgtg     360

aacaagcagt ttaactataa ggacccggtg aacggtgtgg atatcgcgta tatcaaaatc     420

ccgaatgcgg gccagatgca accagtcaag gcgttcaaga ttcataacaa gatttgggtt     480

attccggaac gtgatacctt caccaatccg gaagaaggcg atttaaatcc gccgccagaa     540

gccaaacaag tgccggtgag ctactatgat agcacgtatc ttagcaccga taatgaaaaa 600

gacaattacc tgaagggcgt gaccaagttg ttcgagcgca tctacagtac cgacttaggc 660

cgcatgttgt tgacgagcat cgttcgcggt atcccgttct ggggcggctc gaccattgat 720

accgagttga aagtcattga cacgaactgt atcaatgtta tccaaccgga cggcagttat 780

cgcagcgagg agttaaattt ggtcatcatc ggtccaagcg cagatattat tcagttcgaa 840

tgcaagagct tcggccatga ggtcttgaat ttgacgcgca acggttacgg cagcacccaa 900

tacatccgct ttagcccgga tttcaccttt ggcttcgagg agagcttgga ggtggacacc 960

aacccgctgt taggtgccgg caaattcgca accgacccgg cagtgacgtt ggcgcacgaa 1020

ttgattcatg cgggtcaccg cttatacggt atcgcgatca atccgaatcg cgtctttaaa 1080

gtcaatacca acgcgtacta cgaaatgagc ggcttagagg ttagctttga agaattacgc 1140

accttcggtg gccacgacgc caagttcatc gacagcctgc aggaaaatga gttccgcttg 1200

tactattaca ataaattcaa ggacatcgcg agcaccttaa ataaagcaaa gagcattgtg 1260

ggcaccaccg caagcttgca gtacatgaag aacgtattta aggaaaaata tttgttgtcg 1320

gaggatacca gcgggaaatt cagcgtcgat aagctgaaat tcgacaaatt gtataaaatg 1380

ctgaccgaga tttacaccga ggataacttc gtcaagtttt ttaaggtgtt aaatcgtaag 1440

acctatttaa actttgataa agcggtgttt aaaattaata tcgtgccgaa ggtgaattac 1500

accatctacg atggtttcaa tttacgcaac acgaatctgg cggcgaattt taatggccaa 1560

aacaccgaaa ttaacaacat gaactttacg aagttaaaga atttcacggg cttattcgaa 1620

ttctacaagt tattatgcgt gcgcggcatc attaccagca aggcaggtgc gggcaagtcc 1680

ttggttccgc gtggcagcgc cggcgccggc gcgctcaatg atctgtgtat taaagtcaat 1740

aactgggacc tgttcttcag cccgagcgag gataacttta ccaacgactt aaacaaaggc 1800

gaggagatca cgagcgatac gaacatcgag gcggcggagg aaaatattag cctggacctc 1860

attcagcagt actatctgac gttcaatttt gacaatgagc cggagaacat cagcattgaa 1920

aatctcagca gcgacatcat cggtcagttg gaactgatgc cgaacattga acgctttccg 1980

aacggcaaaa aatatgaact ggacaagtat accatgttcc attacttacg cgcacaggaa 2040

tttgagcacg gcaagagccg cattgcgctg accaatagcg ttaacgaggc cttgttaaat 2100

ccgagccgtg tctacacgtt cttcagcagc gattatgtca aaaaagtgaa caaggcgacc 2160

gaagccgcga tgtttttggg ctgggtcgag caattggttt acgattttac cgacgaaacc 2220

agcgaggtga gcacgaccga caaaattgca gatatcacca tcatcattcc gtacatcggt 2280

ccggcgctca atatcggcaa tatgttatac aaggacgact ttgtgggcgc gctgatcttt 2340

agcggcgcgg ttatcttatt agaattcatc ccggagatcg caatcccggt cttgggcacc 2400

tttgcgttgg tgagctatat cgcgaataaa gtgctcacgg tccaaaccat cgataacgcg 2460

ctcagcaagc gtaatgagaa atgggacgag gtttataagt atatcgtgac caactggtta 2520

gcaaaagtca atacgcagat cgatctcatc cgcaaaaaaa tgaaagaagc cttggaaaat 2580

caagcggagg caaccaaagc catcattaat taccagtata accaatatac cgaagaagaa 2640

aaaaacaata tcaacttcaa tatcgatgat ttgagcagca aactgaacga gagcattaac 2700

aaagcgatga ttaacatcaa caagttcttg aatcaatgca gcgtgagcta tctcatgaac 2760

agcatgatcc cgtatggcgt caaacgcttg gaagattttg acgccagcct gaaagatgcg 2820

ctcctcaagt atatttatga caaccgcggc accctcattg gccaggtgga ccgcttgaag 2880

gataaagtga acaatacgct cagcacggat atcccgttcc agctgagcaa gtacgtcgac 2940

aaccagcgct tactgagcac ctttaccgag tatatcaaga acatcattaa taccagcatc 3000

ctcaacttgc gctatgagag caatcacctg atcgacctca gccgctacgc cagcaagatc 3060

aacatcggca gcaaggtcaa tttcgacccg atcgataaga atcagatcca attgtttaac 3120

ctggaaagca gcaagatcga ggttatcttg aagaacgcga ttgtgtacaa cagcatgtac 3180

gagaacttta gcacgagctt ctggattcgt atcccgaagt atttcaatag cattagcctg 3240

aataacgaat ataccattat caactgcatg gaaaataata gcggctggaa ggtgagctta 3300

aattacggcg agatcatttg gaccttacag gatacccaag aaatcaaaca gcgcgtcgtc 3360

tttaagtata gccagatgat caacatcagc gattacatca accgctggat cttcgtgacc 3420

atcaccaata atcgcttgaa taatagcaag atttacatca atggtcgctt gattgatcaa 3480

aaaccgatca gcaatctcgg taatatccat gccagcaata acatcatgtt taagttagac 3540

ggttgccgcg atacccaccg ctatatctgg atcaagtatt ttaacttatt tgataaggaa 3600

ctcaacgaaa aggaaattaa agacttatat gacaatcaga gcaatagcgg catcctgaag 3660

gatttctggg gcgactacct gcagtacgat aagccgtact atatgttgaa cttgtatgac 3720

ccgaacaaat atgtcgatgt gaacaatgtg ggtattcgtg gctatatgta cttaaagggc 3780

ccgcgtggta gcgtgatgac cacgaatatt tacttaaaca gcagcttata ccgcggcacg 3840

aagtttatta tcaagaagta tgccagcggc aacaaggaca atatcgtccg caacaacgac 3900

cgtgtgtata ttaacgtggt ggtgaagaat aaagagtacc gcttggccac gaatgcgagc 3960

caggcgggcg tggaaaaaat cttgagcgcg ttggagatcc cggacgtcgg caacctcagc 4020

caggttgtgg tgatgaagtc taaaaacgac cagggcatca cgaacaagtg caaaatgaat 4080

ttgcaagata acaacggcaa cgacatcggc tttattggtt ttcaccagtt caataacatc 4140

gccaaactcg tggccagcaa ttggtataac cgccaaattg aacgcagcag ccgcacgctc 4200

ggctgtagct gggagttcat cccggtggac gatggctggg gcgagcgccc gctcggagat 4260

ctggtgccac gcggttccgc gaattcgagc tccgtcgaca agctttggag ccacccgcag 4320

ttcgaaaat aa 4332